# EUROPEAN PATENT SPECIFICATION

(11) **EP 1 302 535 B1**
(45) Date of publication and mention of the grant of the patent: **19.01.2011**
(21) Application number: 01945762.1
(22) Date of filing: 02.07.2001
(51) Int. Cl.: A61L 27/38, C12N 5/071

(54) **HEART MUSCLE-LIKE CELL SHEET, THREE-DIMENSIONAL CONSTRUCT, HEART MUSCLE-LIKE TISSUE AND PROCESS FOR PRODUCING THE SAME**
HERZMUSKEL-ÄHNLICHE ZELLSCHICHT, DREIDIMENSIONALES KONSTRUKT, HERZMUSKEL-ÄHNLICHES GEWEBE UND VERFAHREN ZUR HERSTELLUNG
FEUILLE CELLULAIRE DU TYPE MUSCLE CARDIAQUE, CONSTRUCTION TRIDIMENSIONNELLE, TISSU DU TYPE MUSCLE CARDIAQUE ET PROCEDE DE PRODUCTION ASSOCIE

(30) Priority: 21.07.2000 JP 2000221385
(43) Date of publication of application: 16.04.2003
(60) Divisional application: 10181984.5
(73) Proprietor: Cellseed Inc., Tokyo 160-0022 (JP)
(72) Inventor: OKANO, Teruo, Ichikawa-shi, Chiba 272-0827 (JP); SHIMIZU, Tatsuya, Hachioji-shi, Tokyo 192-0914 (JP); YAMATO, Masayuki, Setagaya-ku, Tokyo 158-0097 (JP); KIKUCHI, Akihiko, Nerima-ku, Tokyo 177-0051 (JP)
(74) Representative: HOFFMANN EITLE
(86) International application number: PCT/JP2001/005722
(87) International publication number: WO 2002/008387

(56) References cited:
- WO-A1-81/01416
- WO-A1-99/66036
- JP-A- 5 192 138
- US-A- 5 543 318
- KIYOTA GOSHIMA: 'Shinkin baiyou-hou no hatten, genjou, mirai, junkan seigyo' CIRC. CONTR. vol. 14, no. 4, 30 December 1993, pages 495 - 500, XP002947549
- MASAYUKI YAMATO ET AL.: 'Ondo outou-sei baiyo-sara kara hi-shinshuu-teki ni kaishuu shita saibou sheet no seika-teki kaiseki to soshiki kougaku e no ouyou' TOKYO JOSHI IKA DAIGAKU SOUGOU KENKYUSHO KIYOU vol. 19, 1998, pages 173 - 174, XP002947550
- DATABASE BIOSIS [Online] TATSUYA SHIMIZU ET AL.: 'Two-dimensional manipulation of cardiac myocyte sheets utilizing temperature-responsive culture dishes augments the pulsatile amplitude', XP002947551 Retrieved from Dialog Database accession no. 200100276118 & TISSUE ENGINEERING vol. 7, no. 2, April 2001, pages 141 - 151
- TATSUYA SIMIZU: 'Cardiac tissue engineering-biomaterial wo mochiita shinkin soshiki no kouchiku' IGAKU NO AYUMI vol. 195, no. 3, 21 October 2000, pages 203 - 204, XP002947552
- AKIHIKO KIKUCHI ET AL.: 'Two-dimensional manipulation of confluently cultured vascular endothelial cells using temperature-responsive poly(N-isoptopylacrylamide)-grafted surfaces' JOURNAL OF BIOMATERIALS SCIENCE POLYMER EDITION vol. 9, no. 12, 1998, pages 1331 - 1348, XP002947553

## Description

### TECHNICAL FIELD

This invention relates to three-dimensional structures comprising myocardium-like cell sheets, and myocardium-like tissues for use in biological, medical and other fields, as well as processes for producing them and therapeutic methods utilizing them.

### BACKGROUND ART

With a view to constructing myocardial tissues *in vitro,* myocardial cells are cultured using three-dimensional supports made of collagen or polylactic acid and many reports on this technique have recently been published. For example, Eschenhagen et al. reported three-dimensional reconstruction in a collagen matrix (Eschenhagen et al., Faseb. J., 11, 683-694, 1997). Carrier et al. reported structures using three-dimensional scaffolds made of polylactic acid (Carrier et al, Biotechnol. Bioeng., 64, 580-589, 1999). Li et al. reported heart grafts prepared in a gelatin mesh by bioengineering techniques (Li et al., J. Thorac. Cardiovasc. Surg., 119, 368-375, 2000). In each of the prior art techniques described in these references, myocardial cells are cultured within a three-dimensional matrix to realize contracting and relaxing behaviors on a culture substrate. However, the cell clumps obtained by these techniques are embedded in a high-molecular weight gel or sponge and using them for a particular purpose, say, for a medical purpose involves the problem of contamination by the high-molecular materials. In addition, no techniques have been available that enable cell sheets to be placed one on another.

Findings have also been reported about the cell culture of non-myocardium tissues such as epidermis. Conventionally, such cell culture has been effected on glass surfaces or on the surfaces of synthetic polymers subjected to a variety of treatments. For example, vessels that are made from polystyrene and which are given surface treatments such as γ-ray irradiation and silicone coating are extensively used in cell culture. Cells cultured and grown in these vessels are peeled off and recovered from their surface by treatment with proteolytic enzymes such as trypsin or with chemicals.

Japanese Patent Publication No. 23191-1990 describes a method of producing transplantable membranes of keratinous tissue by culturing keratinocytes in a culture vessel under conditions where a membrane of keratinous tissue is formed upon the surface of the vessel and enzymatically peeling off the membrane of keratinous tissue. Disclosed specifically is a technique in which 3T3 cells are grown as a feeder layer and piled up in multiple layers and the resulting cellular sheet is recovered with a proteolytic enzyme Dispase. However, the method described in the patent has had the following defects.
(1) Dispase is of bacterial origin and the recovered cellular sheet must be washed thoroughly.
(2) The conditions of treatment with Dispase differ from one cultured cell to another and the treatment requires skill.
(3) The cultured epidermal cells are activated pathologically by Dispase treatment.
(4) Dispase treatment decomposes the extracellular matrix.
(5) As a result, the diseased part to which the recovered cellular sheet has been grafted is susceptible to infection.

Having these drawbacks, the method described in Japanese Patent Publication No. 23191/2000 is difficult to apply to *in vitro* construction of myocardium-like tissues.

Japanese Patent Laid-Open No. 192138/1993 describes a method of culturing skin cells by preparing a cell culture support having a substrate surface coated with a polymer whose upper or lower critical solution temperature in water is 0-80°C, culturing skin cells on the cell culture support at a temperature either below the upper critical solution temperature or above the lower critical solution temperature and thereafter peeling off the cultured skin cells by bringing the temperature to either above the upper critical solution temperature or below the lower critical solution temperature. In this method, temperature change is employed to peel off the cells from the culture substrate coated with the temperature-responsive polymer; however, the method does not permit efficient cell peeling and the obtained cellular sheet has had a lot of structural defects. Hence, the method described in Japanese Patent Laid-Open No. 192138/1993 is also difficult to apply to *in vitro* construction of myocardium-like tissues.

### DISCLOSURE OF THE INVENTION

The present invention has been accomplished in an attempt at solving the aforementioned problems of the prior art. Thus, an object of the invention is to provide a three-dimensional structure of myocardium-like cell sheets made of myocardial tissue cells that retain contracting and relaxing functions, intercellular electrical coupling and orientation. Other object of the invention are to provide a method in which a cultured and grown myocardium-like cell sheet is brought into intimate contact with a polymer membrane and peeled off and recovered from the surface of a support easily and morphologically intact without the need of treatment with an enzyme such as Dispase but by changing the environmental temperature, and producing three-dimensional structures from such cellular sheets.

In order to attain these objects, the present inventors made R&D efforts in which they were reviewed from various angles. As a result, it was found that a cellular sheet having fewer structural defects and furnished with several capabilities that would allow it to function as a myocardium-like tissue *in vitro* could be constructed by a process comprising the steps of culturing myocardial tissue cells on a cell culture support having a substrate surface coated with a temperature-responsive polymer, thereby preparing a myocardium-like cell sheet, treating the sheet to pile up myocardium-like cells by a specified method, thereafter bringing the temperature of the culture solution to either above the upper critical solution point or below the lower critical solution point, bringing the sheet of cultured and piled up cells into intimate contact with a polymer membrane, and peeling the sheet off together with the polymer membrane. It was also found that the cellular sheet could be constructed by a specified method to be given a three-dimensional structure. The present invention has been accomplished on the basis of these findings.

Thus, the present invention provides a three-dimeusional structure of myocardium-like cultured cell sheets made of myocardial tissue cells that retain contracting and relaxing functions, intercellular electrical coupling and orientation.

The invention also provides a three-dimensional structure of myocardium-like cultured cells that retain contracting and relaxing functions, as well as three-dimensional intercellular electrical coupling and orientation, said structure forming tubular cavities of vascular endothelial cells and/or having a single epicardium-like outer cell layer.

The invention further provides a process for producing a three-dimeusional structure of myocardium-like cell sheets, which comprises culturing cells on a cell culture support having a substrate surface coated with a temperature-responsive polymer whose upper or lower critical solution temperature in water is 0∼80°C, and subsequently:
(1) bringing the temperature of the culture solution to above the upper critical solution temperature or below the lower critical solution temperature, and optionally;
(2) bringing the cultured cell sheet into close contact with a polymer membrane; and
(3) peeling the cell sheet off together with the polymer membrane wherein. the myocardium-like cell sheet in close contact with the polymer membrane as obtained by the above-described process is again allowed to adhere to a cell culture support, a cell culture support coated with a temperature-responsive polymer, a polymer membrane or other cellular sheet, the polymer membrane in close contact is thereafter peeled off, and the same procedure is repeated to form a three-dimensional structure of myocardium-like cells.

In addition, the present invention provides a three-dimensional structure that is produced by the above-described processes.

Further according to the invention, the above-described cell three-dimensional structure of myocardium-like cells is buried in the living body to provide a myocardium-like tissue in which tubular cavities are formed of vascular endothelial cells endogenous in said three-dimensional structure of myocardium-like cells and/or the vascular endothelial cells in the tissue around the buried graft are allowed to grow inward and form tubular cavities, thereby forming blood vessels.

In addition, the present invention provides the above-described three-dimensional structure of myocardium-like cells or said myocardium-like tissue that are suitable for use in the treatment of heart disease and other circulatory organ related diseases or digestive organ related diseases.

Further in addition, the present invention provides a method of treating heart disease and other circulatory organ related diseases or digestive organ related diseases using the above-described three-dimensional structure of myocardium-like cells or said myocardium-like tissue.

According to the present invention described above, the myocardium-like cell sheet can be recovered without contamination by a third substance while incurring minimum damage and by superposing a plurality of such myocardium-like sheets, a myocardium-like tissue can be constructed *in vitro* and it is comparable to an *in vivo* myocardial tissue in that it has contracting and relaxing functions, intercellular electrical pacing and orientation. The three-dimensional structure provided by the invention has not been available by the prior art and, hence, the present invention is quite valuable.

### BRIEF DESCRIPTION OF THE DRAWINGS

Fig. 1 shows in micrographs the growth of myocardial cells that were cultured in a temperature-responsive culture dish (grafted in an amount of 2.0 µg/cm²) as compared with the growth in a commercial culture dish not coated with a temperature-responsive polymer.
Fig. 2 shows in charts the motility of myocardial cells that were cultured in a temperature-responsive culture dish (grafted in an amount of 2.0 µg/cm²) as compared with the motility in a commercial culture dish not coated with a temperature-responsive polymer.
Fig. 3 shows in micrographs the morphological change of a sheet of myocardial cells that were subjected to a low-temperature treatment after cultivation on a culture dish to which poly(N-isopropylacrylamide) was grafted.
Fig. 4 illustrates two-dimensional manipulation (manipulation method (1)) for producing a myocardial cell sheet.
Fig. 5 shows in micrographs the morphological changes that occurred to the cytoskeleton (F-actin) and cell nuclei (Nuclei) during manipulation method (1).
Fig. 6 shows in charts the motility of myocardial cells that correspond to the three states shown in Fig. 5.
Fig. 7 illustrates a method (manipulation method (2)) of producing a three-dimensional structure consisting of two myocardium-like cell layers by performing manipulation method (1) twice.
Fig. 8 shows in micrographs cross sections of a myocardial cell sheet and a three-dimensional structure that were produced by manipulation methods (1) and (2), respectively.
Fig. 9 illustrates a method (manipulation method (3)) in which the polymer membrane used in manipulation method (1) was replaced by a polymer mesh and the myocardial cell sheet as peeled off was turned over together with the polymer mesh and fixed in a culture dish.
Fig. 10 illustrates a method (manipulation method (4)) in which the polymer mesh was preliminarily fixed on a separate culture dish, to which a myocardial cell sheet was subsequently transferred in accordance with manipulation method (1).
Fig. 11 shows in micrographs a surface of a myocardial cell sheet produced by manipulation method (3) (the upper picture) and a cross section of the same cell sheet after staining with H.E. (the lower picture).
Fig. 12 shows in micrographs the myocardial cell sheet produced by manipulation (3) (the upper pictures), as well as showing in charts the motility of the same cell sheet.
Fig. 13 shows in micrographs cross sections of a three-dimensional structure of myocardium-like cells that was produced by manipulation method (3) and stained with hematoxylin-eosin.
Fig. 14 is a pair of micrographs, the upper picture of which is a cross section of a three-dimensional structure of cultured myocardium-like cells that was produced by manipulation method (3) and stained with hematoxylin-eosin.
Fig. 15 illustrates a method (manipulation method (5)) of producing a three-dimensional structure of two myocardium-like cell layers by an improvement of manipulation method (3).
Fig. 16 shows in micrographs a surface of the three-dimensional structure of myocardium-like cells that was produced by manipulation method (5) (the upper picture) and a cross section of the same structure after staining with H.E. (the lower picture).
Fig. 17 illustrates the transmission of an electrical stimulus from one myocardium-like cell sheet to another that was in partial overlap with the first sheet.
Fig. 18 shows in charts the electrocardiographic complexes from a host rat heart and a grafted cell structure that were used in Example 2.
Fig. 19 is a micrograph showing an Azan-stained tissue slice around the cell structure grafted into a rat in Example 2.
Fig. 20 is a micrograph showing a Factor VIII-stained tissue slice around the cell structure grafted into a rat in Example 2.

### BEST MODE FOR CARRYING OUT THE TNVENTION

The myocardial tissue cells to be used in the invention are not limited in any particular way as long as they are obtained from the living heart and usually among them are myocardial cells, vascular endothelial cells and fibroblast cells. By culturing these cells, one can produce a monolayered myocardium-like cultured cell sheet which is given a three-dimensional shape by a specified method, producing a three-dimensional structure of myocardium-like cultured cells.

The myocardium-like cultured cell sheet and the three-dimensional structure described herein are free from any damage that would otherwise be caused by treatment with proteolytic enzymes such as Dispase and trypsin during culture. Therefore, the cell sheet and three-dimensional structure that are peeled off from the substrate can be recovered as adequately strong cell clumps that retain intercellular protein and they keep the capabilities characteristic of myocardial cells such as contracting and relaxing functions, intercellular electrical coupling and orientation. The three-dimensional structure also presents with several characteristic cell arrangements that resemble living tissues as exemplified by the formation of epicardium composed of connective tissue and the formation of tubular cavities from vascular endothelial cells. Described specifically, if a customary proteolytic enzyme such as trypsin is used, intercellular desmosome structures and the cell-to-substrate protein resembling the basal lamina are seldom kept intact and, hence the cells become separate individually as they are peeled off from the substrate. Among the applicable proteolytic enzymes, Dispase which destroys practically all of the cell-to-substrate protein resembling the basal lamina is known to be capable of allowing the cells to be peeled off from the substrate while keeping 10∼60% of desmosome structures intact. However, the obtained cellular sheet has only weak strength. In contrast, desmosome structures and basal lamina-like protein each remain by at least 80% in the cellular sheet described herein and the above-described various advantages can be obtained.

The temperature-responsive polymer used to coat the substrate of the cell culture support has an upper or lower critical solution temperature in aqueous solution which is generally in the range of 0°C ∼ 80°C, preferably 20°C ∼ 50°C. If the upper or lower critical solution temperature exceeds 80°C, cells may potentially die and this is not preferable. If the upper or lower critical solution temperature is lower than 0°C, the cell growth rate will usually drop by an extreme degree or cells will die, which is not preferable, either.

The temperature-responsive polymer to be used in the invention may be a homopolymer or a copolymer. Exemplary polymers are described in Japanese Patent Laid-Open No. 211865/1990. Specifically, they are obtained by homo- or co-polymerization of the following monomers. Useful monomers include, for example, (meth)acrylamide compounds, N-(or N,N-di)alkyl-substituted (meth)acrylamide derivatives and vinyl ether derivatives; in the case of copolymers, any two or more of these monomers may be employed. Further, those monomers may be copolymerized with other monomers, or one polymer may be grafted to another or two polymers may be copolymerized or a mixture of polymer and copolymer may be employed. If desired, polymers may be crosslinked to an extent that will not impair their inherent properties.

The substrate which is to be provided with coatings may be of any types including those which are commonly used in cell culture, as exemplified by glass, modified glass and compounds such as polystyrene and poly(methyl methacrylate), as well as substances that can generally be given shape, for example, polymer compounds other than those listed above and ceramics.

The method of coating the support with the temperature-responsive polymer is not limited in any particular way and it may be in accordance with the disclosure in Japanese Patent Laid-Open No. 211865/1990. Specifically, such coating can be realized by subjecting the substrate and the above-mentioned monomer or polymer to either one of electron beam (EB) exposure, irradiation with γ-rays, irradiation with uv rays, plasma treatment, corona treatment and organic polymerization reaction, or other techniques such as physical adsorption as achieved by coating application and kneading may be adopted.

In the present invention, myocardial cells are cultured on the cell culture support (e.g. cell culture dish) that has been prepared as described above. The temperature of the medium is not limited to any particular value; if the aforementioned polymer forming the coat on the substrate surface has an upper critical solution temperature, the temperature of the medium may be lower than such upper critical solution temperature; if said polymer has a lower critical solution temperature, the temperature of the medium may be higher than such lower critical solution temperature. Of course, culturing is inappropriate if it is in a low-temperature range where cultured cells do not grow or in a high-temperature range where cultured cells die. Culture conditions other than temperature may be as adopted in the conventional techniques and are not limited in any particular way. For example, the medium to be used may be one that is supplemented with sera such as known fetal calf serum (FCS); alternatively, it may be a serum-free medium or one which is not supplemented with any sera.

In the method of the invention, the culture time may be set in accordance with the aforementioned procedure to a value that suits the specific object of using the myocardium-like cell sheet or three-dimensional structure. In order to peel off and recover the cultured myocardium-like cell sheet or three-dimensional structure from the support material, the cultured cell sheet or three-dimensional structure is either held as it is or optionally brought into close contact with the polymer membrane and the temperature of the support material to which the cells are adherent is brought to above the upper critical solution temperature of the polymer with which the support's substrate is coated or below its lower critical solution temperature, whereby the cultured cell sheet or three-dimensional structure can be peeled off from the substrate either independently or together with the polymer membrane which has been brought into intimate contact with them. Peeling of the myocardium-like cell sheet or three-dimensional structure can be performed not only in the culture solution used to culture the myocardial cells but also in other isotonic solutions; a suitable solution can be chosen in accordance with a specific object. Examples of the polymer membrane that can optionally be used to achieve close contact with the cell sheet or the three-dimensional structure include polyvinylidene difluoride (PVDF), polypropylene, polyethylene, cellulose and its derivatives, as well as chitin, chitosan, collagen, papers such as Japanese paper, polyurethane, net- or stockinet-like polymeric materials such as Spandex, etc. Using net- and stockinet-like polymer materials, one can produce cell sheets and three-dimensional structures having more degrees of freedom and further enhanced contracting and relaxing capabilities. The method of producing the three-dimensional structure of myocardial cells according to the invention is not limited to any particular types; an exemplary method is using the myocardial cultured cell sheet in close contact with the above-mentioned polymer membrane. The following methods may be given as specific examples.
(1) The cellular sheet in intimate contact with the polymer membrane is allowed to adhere to the cell culture support and thereafter a medium is added to peel off the polymer membrane from the cellular sheet; then, a cellular sheet in intimate contact with another polymer membrane is allowed to adhere to the first cellular sheet; this process is repeated to form a pile of cellular sheets.
(2) The cellular sheet in intimate contact with the polymer membrane is turned over and fixed on the cell culture support such that the polymer membrane contacts the support; another cellular sheet is allowed to adhere to the first cellular sheet; thereafter, a medium is added to peel off the polymer membrane from the second cellular sheet, to which yet another cellular sheet is allowed to adhere; this process is repeated to form a pile of cellular sheets.
(3) Two cellular sheets each in intimate contact with the polymer membrane are allowed to adhere to each other.

In order to peel off and recover the myocardium-like cell sheet and three-dimensional structure in high efficiency, tapping on the cell culture support or shaking it gently, agitating the medium with a pipette and other methods may be employed either alone or in combination. In addition, the myocardium-like cell sheet may optionally be washed with an isotonic solution and the like to be peeled off and recovered.

The myocardium-like cell sheet or three-dimensional structure that have been peeled off from the substrate may be stretched in a specified direction to produce an oriented cell sheet or three-dimensional structure. The stretching method is not limited at all and an example is the use of a tensile apparatus such as Tensilon or simply pulling the sheet or structure with tweezers. By being oriented, the cellular sheet or three-structure can be given directionality in their movement. Since this enables the cellular sheet or three-dimensional structure to be placed over a specified organ while assuring compliance to its movement, they can be applied to organs with high efficiency.

The three-dimensional structure produced by the above-described methods have not been attainable by the prior art methods. The produced three-dimensional structure retains the basal lamina that has been cut off in the prior art, so no matter where in the living body they are buried, including arms, shoulders, legs and any other non-heart organs and parts, three-dimensional structure adhere sufficiently viably to the surrounding tissue that they will pulsate at every site where they were grafted. The probable reason for this would be as follows: as soon as the buried three-dimensional structure adheres viably to the living tissue,it starts contracting and relaxing to suffer hypoxia and in order to compensate for this, vascular endothelial cells positively grow inward from the living tissue to form blood vessels, whereby not only oxygen but also nutrients are adequately supplied via blood. In this way, the three-dimensional structure that has been buried in the living body contribute to *in vivo* formation of a myocardium-like tissue. It is therefore held to have much promise in grafting and other clinical applications. Specifically, if the three-dimensional structure or myocardium-like tissue of the invention is grafted to a site of weakened contractile force in the heart, they will prove to be effective tools in treating heart disease such as myocardial infarction; alternatively, they may be applied to the surrounding of blood vessels in order to improve blood circulation, thus proving to be useful tools in treating serious abscess, severe stiff neck and dysfunction of the aorta.

Note that the cell culture support to be employed in the method of the invention can be used over and again.

### Examples

On the pages that follow, the present invention is described in greater detail with reference to examples, which are by no means intended to limit the invention.

### Example 1

### (Materials and apparatuses used)

1. Cell: Primary chick embryo myocardial cells were collected and cultured by ordinary procedures (see Cardiovasc. Res., 41, 641 (1999)).
2. Culture dish: A temperature-responsive culture dish (grafted in an amount of 2.0 µg/cm²). As a control, a commercial culture dish (Falcon 3001) not coated with a temperature-responsive polymer was used.
3. Medium: There was used a medium consisting of 6% FBS, 40% 199 medium, 0.2% penicillin-streptomycin, 2.7 mM glucose and 54% physiological saline (116 mM NaCl, 1.0 mM NaH₂PO₄, 8 mM MgSO₄, 1.18 mM KCl, 0.87 mM CaCl₂ and 26.2 mM NaHCO₃).
4. Culture: The primary chick embryo myocardial cells were plated onto a 35-mm culture dish at a cell density of 0.5 x 10⁶ cells/cm².
5. Other conditions were in accordance with the ordinary procedures.
6. Observing cells under microscope: A phase-contrast microscope (ET300 of Nikon) and a fluorescence microscope (DMIRB of Leica) were used.
7. Method of measuring contracting and relaxing behaviors: Pictures taken with a CCD camera (RS-170 of COHU) were subjected to image analysis.

### (Method)

The following specific method was employed to prepare myocardium-like cell sheets.

From a 10-day chick embryo, myocardial cells were isolated using trypsin and cultured on a temperature-responsive culture dish, namely, a culture dish to which poly(N-isopropylacrylamide) (PIPAAm) had been grafted. In order to reduce the adhesion to cells, meshes of various polymers were used as supports and subjected to low-temperature treatment (20°C). The myocardial cell sheet adhering to the mesh was detached from the culture dish, on which it was turned over and cultured in a free state. The mesh adhering to the myocardial cell sheet was piled up on another myocardial cell sheet that had been cultured on a temperature-responsive culture dish; after low-temperature treatment, the two myocardial cell sheets were turned over together with the mesh and cultured. Cell morphology was visualized by taking the images of tissue slices (stained with H.E. and Azan). The contracting and relaxing functions of cardiac cells were quantitated with an image analyzer using images that were recorded with a VTR via a CCD camera connected to a microscope. While the method of quantification is not limited in any particular way, an example may be such that visible low and high density areas are specified within the cytoplasm and their movements are monitored with the image analyzer and their loci determined.

### (Results)

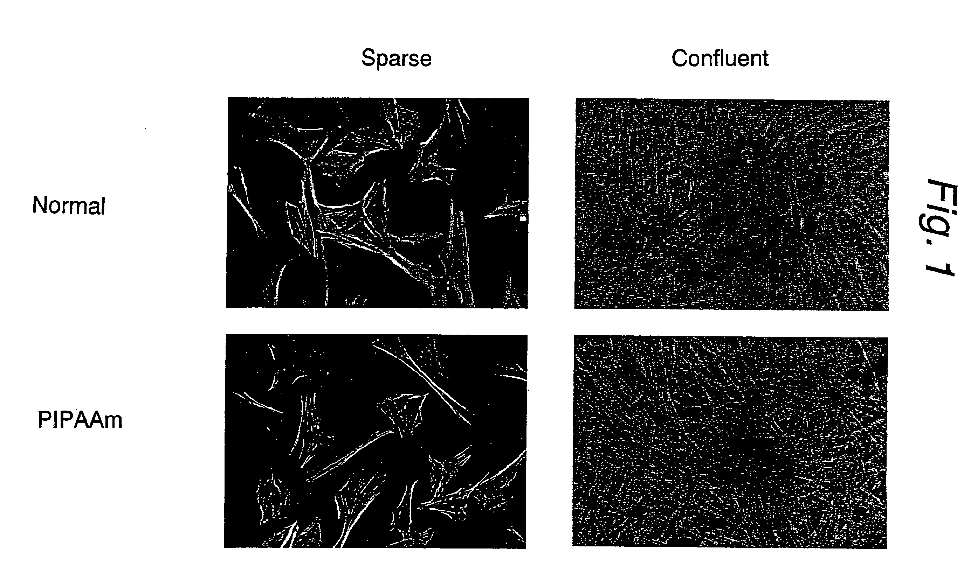

Fig. 1 shows in micrographs the growth of myocardial cells that were cultured in the temperature-responsive culture dish (grafted in an amount of 2.0 µg/cm²) as compared with the growth in the commercial culture dish not coated with a temperature-responsive polymer. In Fig. 1, Normal refers to the case where culture was effected on the commercial culture dish and PIPAAm refers to the case of culture on the temperature-responsive culture dish. Sparse indicates sparse growth of myocardial cells and Confluent indicates confluency of myocardial cells. It can be seen from Fig. 1 that myocardial cells were as confluent on the poly(N-isopropylacrylamide) grafted culture dish as on the normal commercial culture dish.

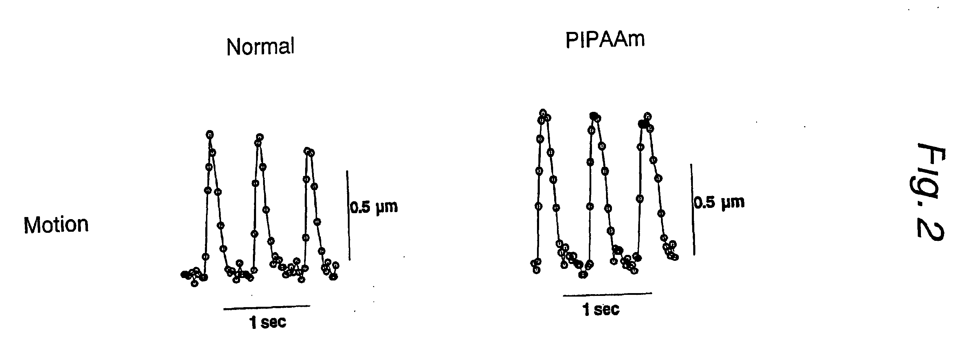

Fig. 2 shows in charts the motility of myocardial cells cultured in the temperature-responsive culture dish (grafted in an amount of 2.0 µg/cm²) as compared with the motility in the commercial culture dish not coated with a temperature-responsive polymer. In Fig. 2, Normal refers to the case where culture was effected on the commercial culture dish and PIPAAm refers to the case of culture on the temperature-responsive culture dish. Motion shows the result of measurement of cell amplitude with the image analyzer, which reflects the contraction and relaxation of myocardial cells. It can be seen from Fig. 2 that the myocardial cells cultured on the poly(N-isopropylacrylamide) grafted culture dish had comparable contracting and relaxing functions to the myocardial cells cultured on the normal commercial culture dish. The difference is the myocardial cells cultured on the poly(N-isopropylacrylamide) grafted culture dish varied over a smaller range since they adhered to the dish more firmly.

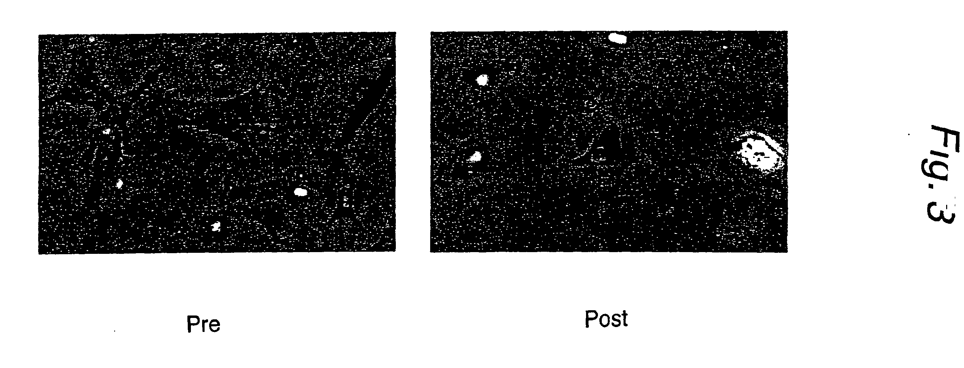

Fig. 3 shows in micrographs the morphological change of myocardial cells that were subjected to a low-temperature treatment after cultivation on the poly(N-isopropylacrylamide) grafted culture dish. In Fig. 3, Pre shows the morphology of myocardial cells before the low-temperature treatment and Post, after the low-temperature treatment. It is clear from Fig. 3 that the cells on the poly(N-isopropylacrylamide) grafted culture dish could be peeled off by the low-temperature treatment.

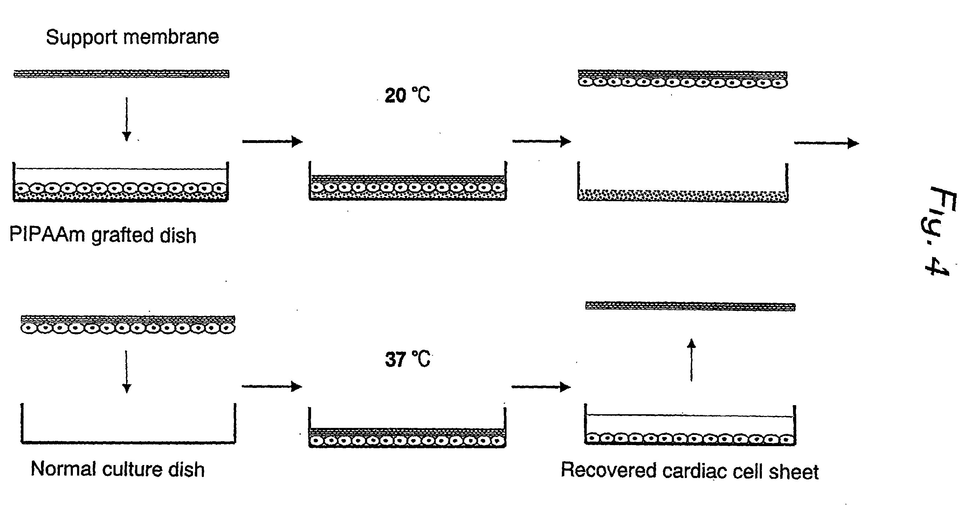

Fig. 4 illustrates two-dimensional manipulation for producing a myocardial cell sheet and this method is hereunder referred to as manipulation method (1). The first step in this method is culturing myocardial cells on the polyisopropylacrylamide grafted culture dish. After reaching confluency, the cells were subjected to low-temperature treatment in intimate contact with a polymer membrane (polyvinylidene difluoride), peeled off from the culture dish and transferred into another culture dish. In Fig. 4, Support membrane refers to the polymer membrane, PIPAAm grafted dish refers to the poly(N-isopropylacrylamide) grafted culture dish, Normal culture dish refers to the normal commercial culture dish, and Recovered cardiac cell sheet refers to the recovered myocardial cell sheet.

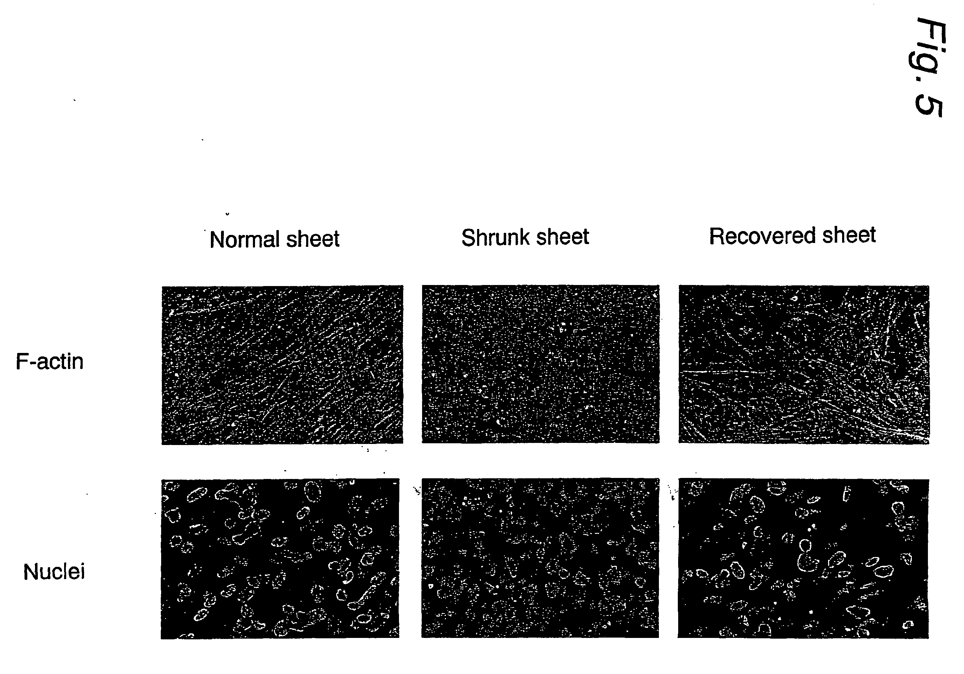

Fig. 5 shows in micrographs the morphological changes that occurred to the cytoskeleton (F-actin) and cell nuclei (Nuclei) during manipulation method (1). In Fig. 5, Normal sheet refers to the cells cultured on the poly(N-isopropylacrylamide) grafted culture dish, Shrunk sheet refers to the cells that rolled into themselves after the sheet was peeled off from the dish, and Recovered sheet refers to the myocardial cell sheet that was allowed again to adhere to a cell culture support after being recovered together with the polymer membrane. It is clear from Fig. 5 that the cell sheet transferred into the separate culture dish (as identified by Recovered sheet in Fig. 5) could reproduce the morphology of the cells before transfer.

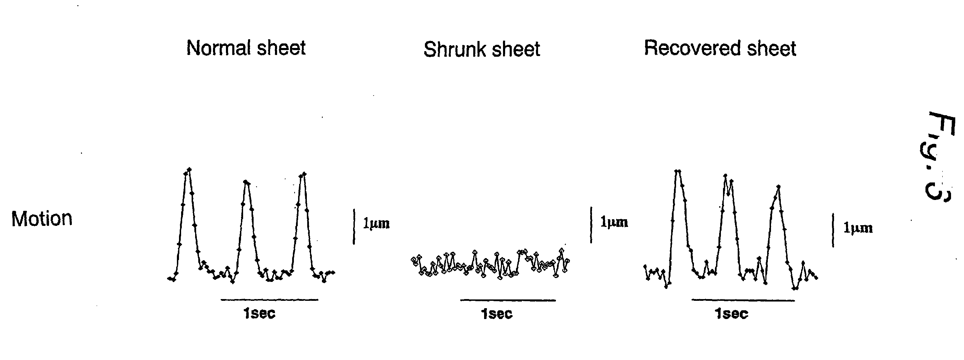

Fig. 6 shows in charts the motility of myocardial cells that correspond to the three states shown in Fig. 5. Motion shows the result of measuring the cell amplitude with the image analyzer which reflects the contraction and relaxation of myocardial cells. Fig. 6 supports that the contracting and relaxing functions of myocardial cells were maintained both before and after performing manipulation method (1) (compare Normal sheet with Recovered sheet). The cell sheet that was not peeled off together with the polymer membrane could be peeled off from the cell culture support by the low-temperature treatment; however, the cell sheet shrank and rolled into itself, with the cells failing to exhibit the desired contracting and relaxing functions.

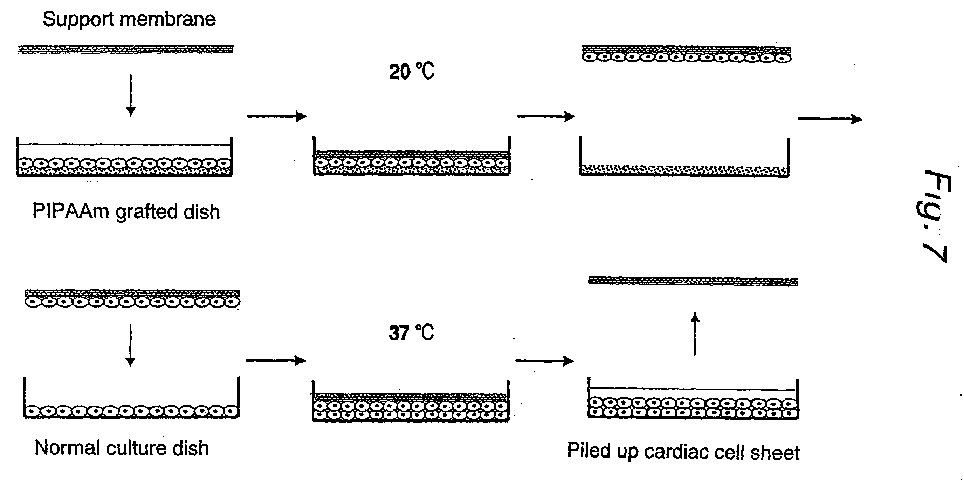

Fig. 7 illustrates a method of producing a three-dimensional structure consisting of two myocardial cell layers by performing manipulation method (1) twice and the method is hereunder referred to as manipulation method (2). In manipulation method (2), the cell sheet in intimate contact with the polymer membrane is allowed to adhere to the cell culture support and thereafter a medium is added to peel off the polymer membrane from the cell sheet, which is allowed to adhere to another cell sheet in intimate contact with polymer membrane; this process is repeated to pile up cell sheets.

In Fig. 7, Support membrane refers to the polymer membrane, whereas PIPAAm grafted dish and Normal culture dish have the same meanings as defined before, and Piled up cardiac cell sheets refers to the myocardial cell sheets in two layers.

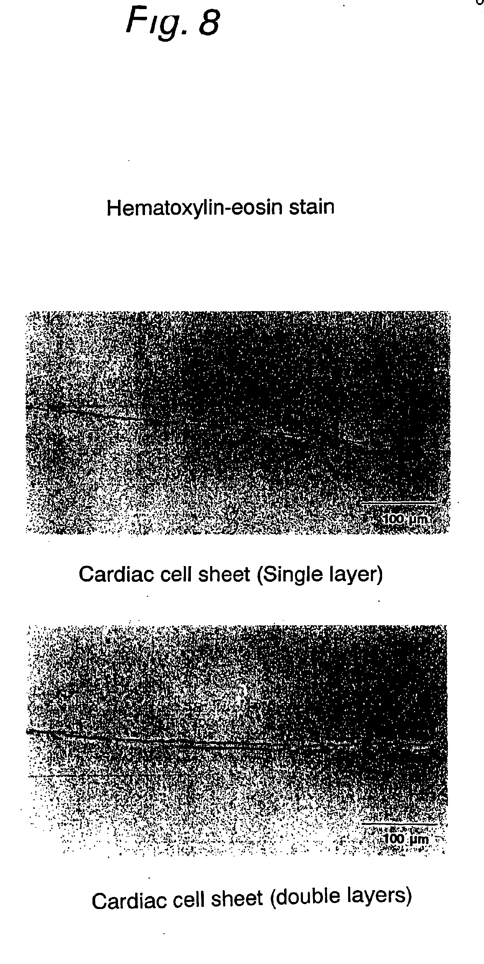

Fig. 8 shows in micrographs cross sections of myocardial cell sheets that were produced by manipulation methods (1) and (2). Both types of myocardial cell sheet were stained with hematoxylin-eosin. In Fig. 8, Single layer shows the appearance in cross section of the myocardial cells (as single layer) that were produced by manipulation method (1) and Double layers shows the appearance in cross section of the myocardial cells (as dual layer) that were produced by manipulation method (2). Hematoxylin-eosin stain means that those cell sheets were stained with hematoxylin-eosin.

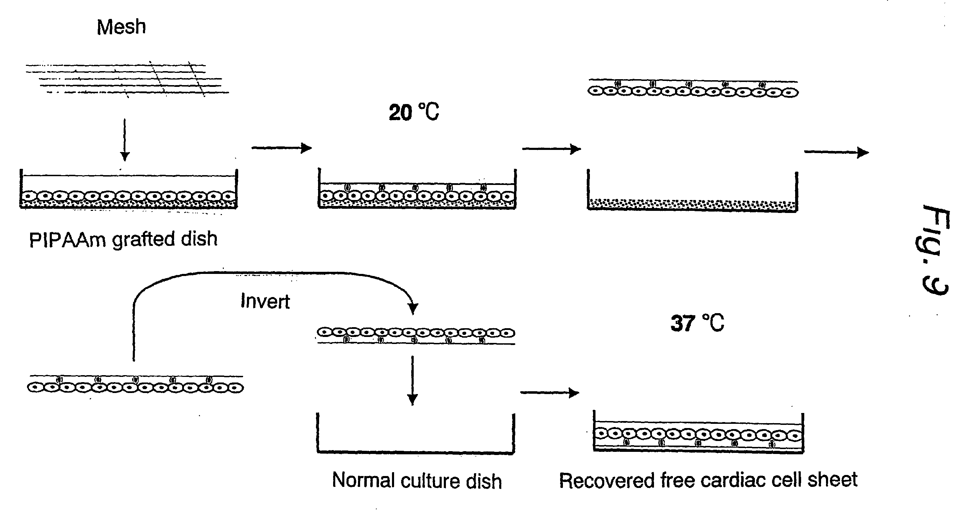

Fig. 9 illustrates a method in which the polymer membrane used in manipulation method (1) was replaced by a polymer mesh and the myocardial cell sheet as peeled off was turned over together with the polymer mesh and fixed in a culture dish and the method is hereunder referred to as manipulation method (3). According to manipulation method (3), the mesh used as the polymeric material in contact with the myocardial cell sheet helped enhance its contracting and relaxing functions. In Fig. 9, Mesh refers to the polymeric mesh, whereas PIPAAm grafted dish and Normal culture dish have the same meanings as defined before, Recovered free cardiac cell sheet refers to the recovered free myocardial cell sheet, and Invert means turning over the peeled myocardial cell sheet in the culture dish together with the polymer mesh.

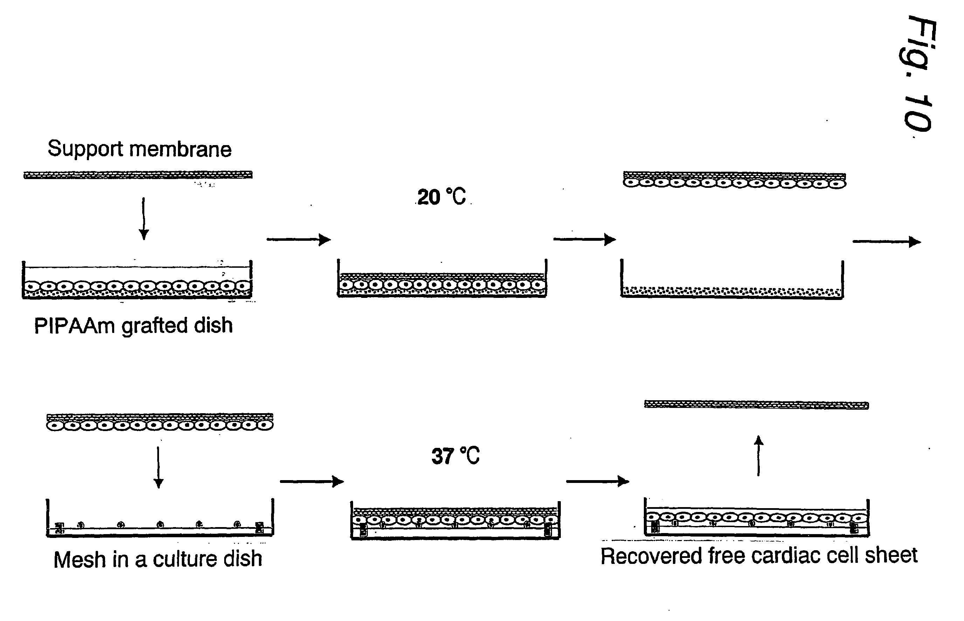

Fig. 10 illustrates a method in which the polymer mesh was preliminarily fixed on a separate culture dish, to which a myocardial cell sheet was subsequently transferred in accordance with manipulation method (1) and the method is hereunder referred to as manipulation method (4). In Fig. 10, Support membrane, PIPAAm grafted dish and Recovered free cardiac cell sheet have the same meanings as defined before, and Mesh in a culture dish refers to the mesh fixed on the culture dish.

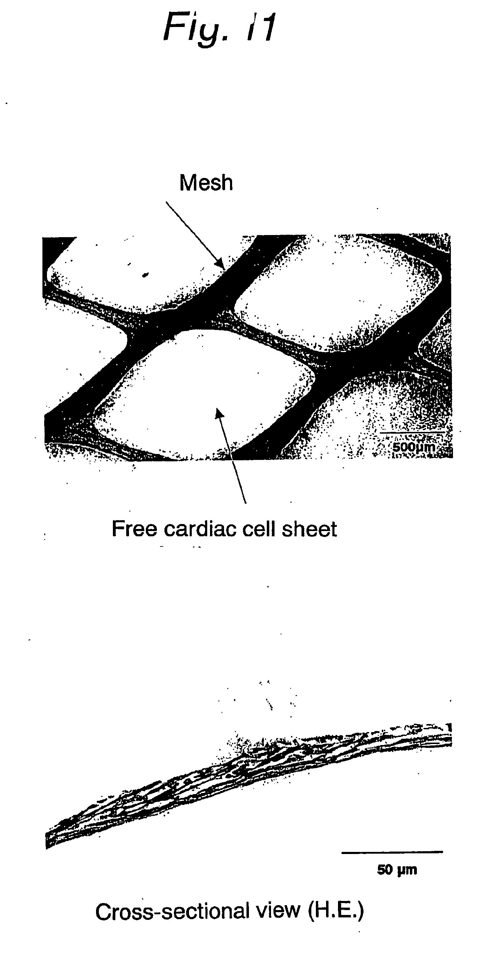

Fig. 11 shows in micrographs a surface of a myocardial cell sheet produced by manipulation method (3) (the upper picture) and a cross section of the same cell sheet after staining with H.E. (the lower picture). In Fig. 11, Mesh has the same meaning as before, Free cardiac sheet refers to the free myocardial cell sheet, and Cross-sectional view (H.E.) shows a cross section of the H.E. stained sheet.

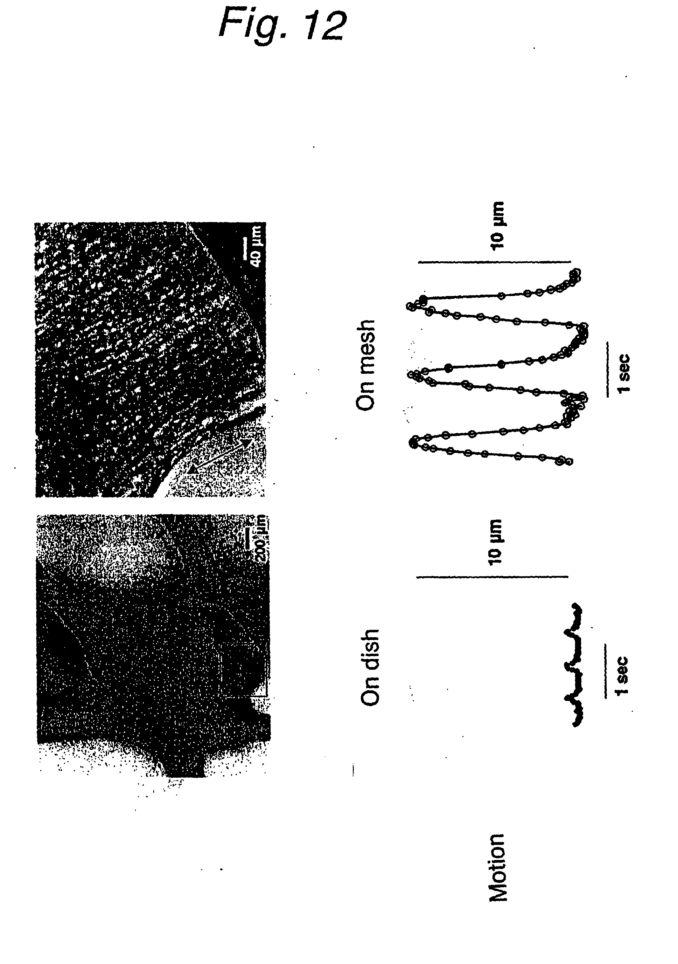

Fig. 12 shows in micrographs the myocardial cell sheet produced by manipulation method (3) (the upper pictures), as well as showing in charts the motility of the same cell sheet. In Fig. 12, Motion has the same meaning as defined before, On dish refers to the cell sheet on a culture dish, and On mesh refers to the cell sheet on the mesh. It can be seen from Fig. 12 that the support in mesh form contributed to increasing the changes in motility of myocardial cells.

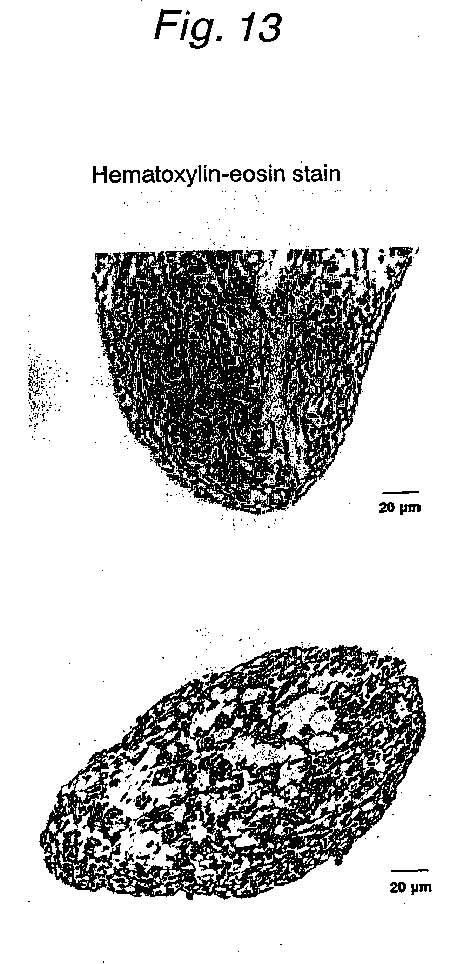

Fig. 13 shows in micrographs cross sections of a cultured myocardial cell sheet that was produced by manipulation method (3) and stained with hematoxylin-eosin.

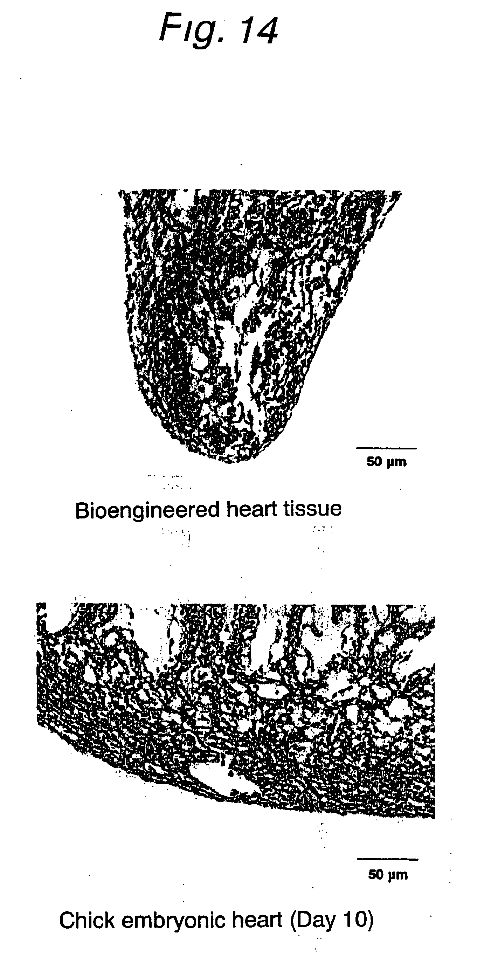

Fig. 14 shows in micrographs cross sections of the myocardial tissue in a cultured myocardial cell sheet that was produced by manipulation method (3) and stained with hematoxylin-eosin (the upper picture) and a myocardial tissue from chick embryo at day 10 (the lower picture). It can be seen from Fig. 14 that the myocardial tissue obtained by the method of the invention has a very similar structure to the *in vivo* tissue.

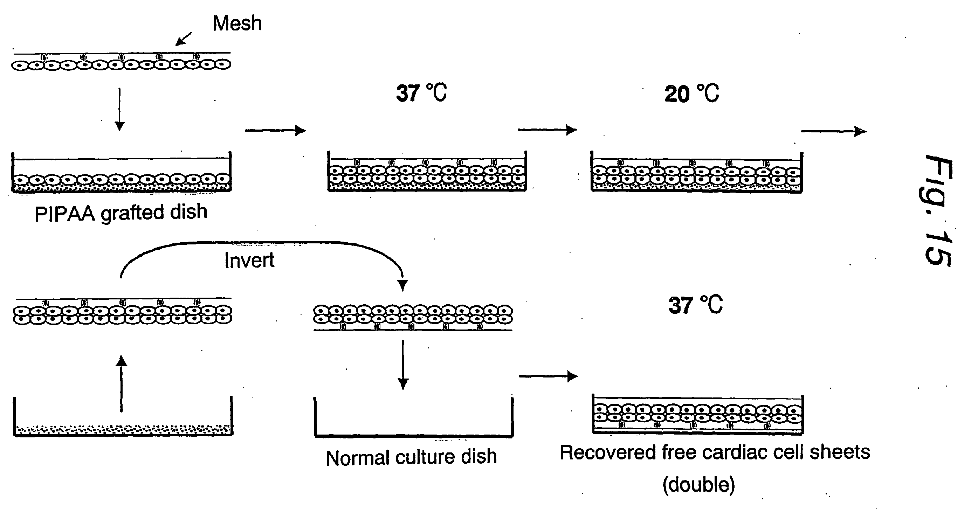

Fig. 15 illustrates a method of producing a two-layered myocardial cell sheet by an improvement of manipulation method (3) and the method is hereunder referred to as manipulation method (5). In manipulation method (5), a stocking is used as a polymer mesh. In Fig. 15, Mesh, PIPAAm grafted dish, Invert and Normal culture dish have the same meanings as defined before. Recovered free cardiac cell sheets (Double) refers to the recovered free myocardial cell sheets (in two layers).

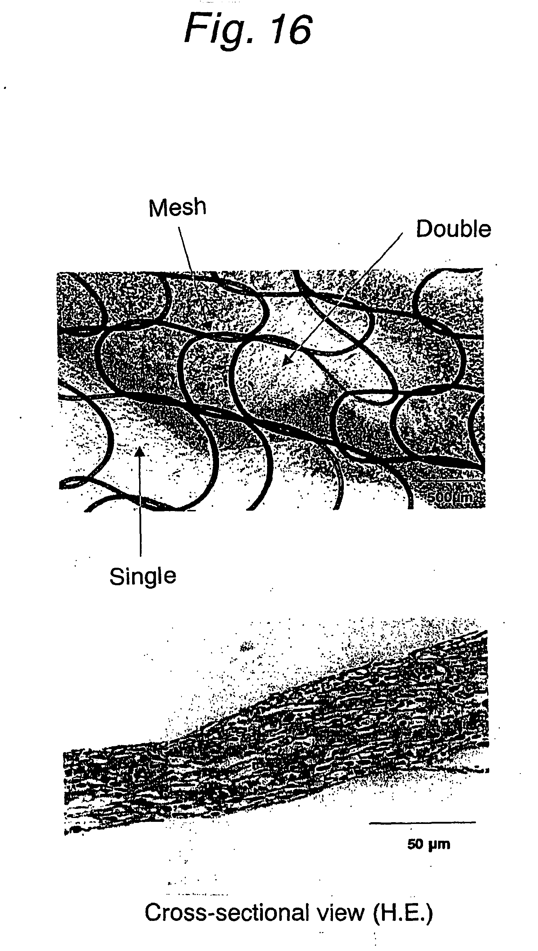

Fig. 16 shows in micrographs a surface of the dual layered myocardial cell sheet that was produced by manipulation method (5) (the upper picture) and a cross section of the same sheet after staining with H.E. (the lower picture). In Fig. 16, Mesh and Cross-sectional view (H.E.) have the same meanings as defined before. Single in the upper picture represents the first layer and Double, the second layer.

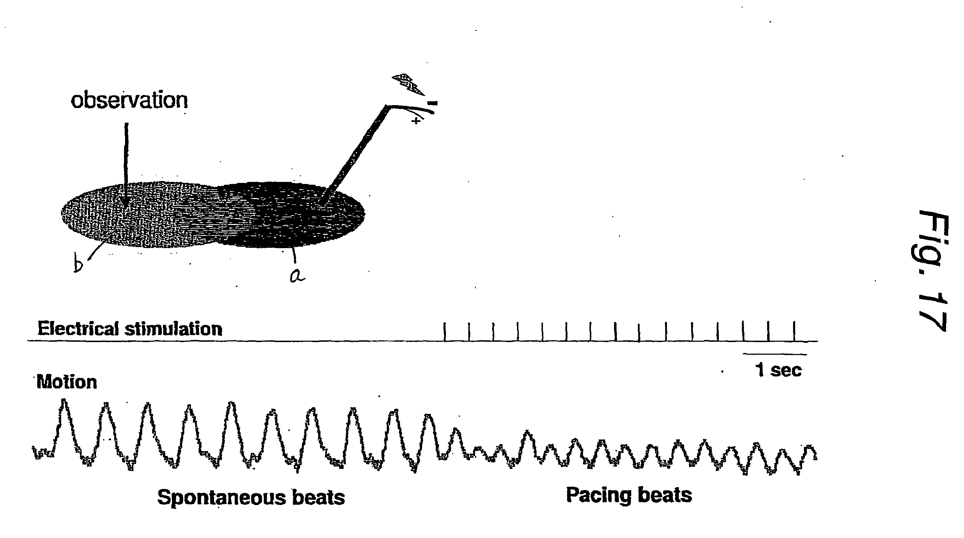

Fig. 17 illustrates the transmission of an electrical stimulus from one myocardium-like cell sheet (on the right side of the Figure and indicated by a) to another cell sheet that was in partial overlap with the first sheet (on the left side of the Figure and indicated by b). Since Fig. 17 is a horizontally elongated drawing, the relative vertical positions are shown as rotated counterclockwise through 90 degrees. In Fig. 17, Electrical stimulation means pulsed electrical stimuli sent from the cell sheet on the right side and the vertical bars appearing in the latter half (on the right side) of the line extending from under Electrical stimulation represent the pulsed stimuli. The applied stimuli transmitted to the cell sheet on the left side were measured by observing the above-defined cell amplitude with an image analyzer (as indicated by observation in Fig. 17). In Fig. 17, Motion refers to the result of cell amplitude measurement with the image analyzer and Spontaneous beats refers to the behavior of cells that were given the electrical stimuli. Obviously, the electrical stimuli applied to one cell sheet increased the contracting and relaxing rhythm of the other cell sheet. This means that the myocardium-like cell sheets produced by the present invention retain intercellular electrical coupling not only within one sheet but also between two superposed sheets.

### (Discussion)

Example 1 and the results shown in Figs. 1-17 revealed the following.
(1) The myocardial cell sheet on mesh and its three-dimensional structure contracted and relaxed by greater extent than when they were fixed on the inner surface of a culture dish. This would be because the individual cells acquired more degrees of freedom by being detached from the culture dish.
(2) When myocardial cell sheets were piled up to produce a three-dimensional structure, two sheets were found to adhere firmly to each other on a tissue slice. In addition, the myocardial cell sheets in pile pulsated in general synchronism, suggesting electrical coupling between the two sheets. When the entire myocardial cell sheet was contracted in one direction on the mesh, the myocardial cells aligned to form three-dimensional bundles in a direction perpendicular to the first direction; as a result, the myocardial cells became oriented and pulsed in the direction of major axis.
(3) As a result of prolonged (5-day) culture, a single epicardium-like cell layer was observed around bundles of myocardial cells on the cross section of tissue slice.

### Example 2

Myocardial cells were isolated from a newborn rat with collagenase and plated onto the same temperature-responsive culture dish as used in Example 1. Culture was performed for 4 days on the same medium and under the same conditions as in Example 1 and the confluent myocardial cell sheet was peeled off by low-temperature treatment. Two peeled myocardium-like cell sheets were placed one on the other by pipetting in the culture solution. This was done without using a polymer membrane and the myocardial sheets as they shrank after peeling were immediately placed in superposition. In about 20 minutes, the two sheets adhered firmly to each other. The obtained three-dimensional structure was transplanted into a dorsal subcutaneous tissue of a nude rat (rat with immunodeficiency F344) by a conventional method.

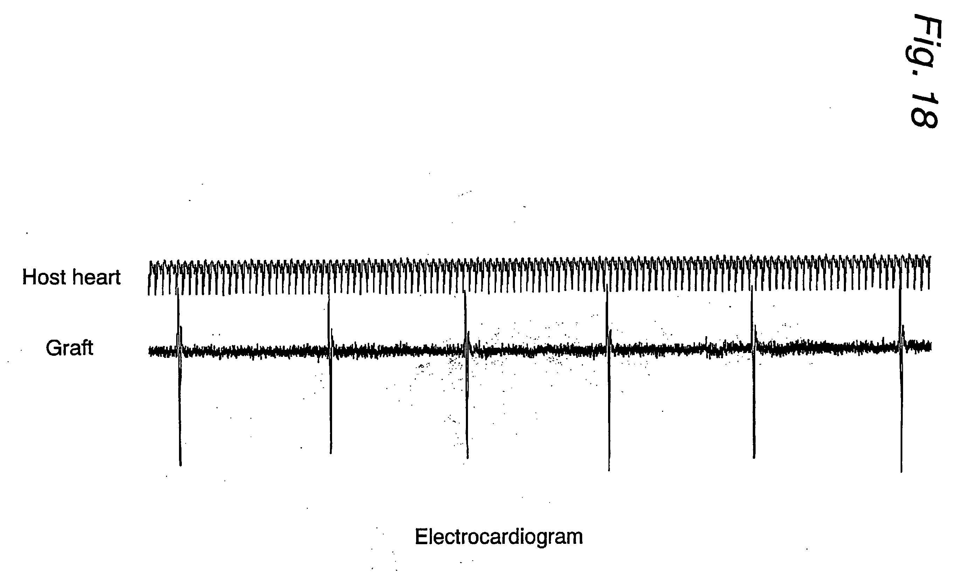

As Fig. 18 shows, an electrocardiographic complex was observed from the rat's body surface immediately after transplantation; it was independent of the pulsation of the rat's host heart and originated from the grafted cell structure. Three weeks after transplantation, the graft site was incised and the pulsation of the grafted cell structure and the neogenesis of blood vessels in that structure which grew from the living tissue were both recognized by the naked eye.

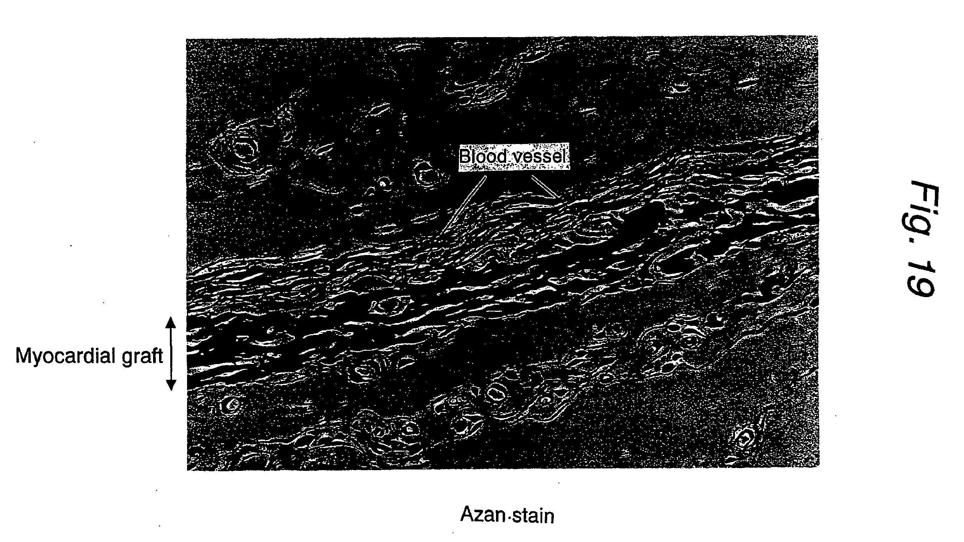

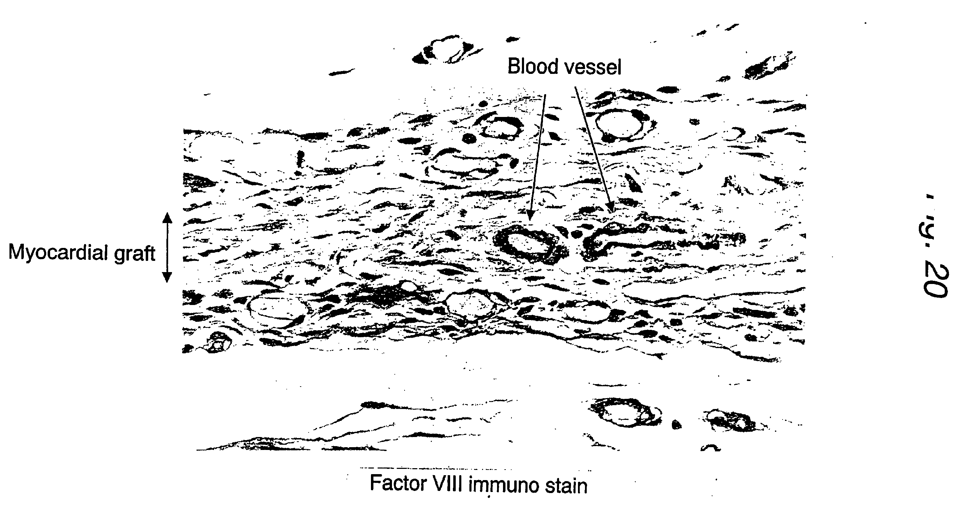

Fig. 19 is a micrograph showing a H.E. and Azan-stained tissue slice around the grafted cell structure. The grafted cell structure was a muscular tissue and stained red whereas the subcutaneous tissue into which it was grafted was a connective tissue and stained blue. Redstained blood vessels are recognized in the grafted cell structure, indicating the presence of red blood cells in the vessels. The formation of blood vessels in the grafted cell structure could also be verified from Fig. 20 which is a micrograph showing the result of Factor VIII immunostain characterized by selective staining of vascular endothelial cells.

From the foregoing, it is clear that a structure resembling the myocardial tissue and neogenesis of blood vessels in the grafted cell structure also occurred on the tissue slice.

### INDUSTRIAL APPLICABILITY

According to the present invention, myocardial cell sheets can be cultured three-dimensionally to construct a myocardium-like tissue. Therefore, the invention holds great promise in clinical applications and is extremely useful in medical and biological fields, particularly in cell engineering and medical engineering.

## Claims

1. A process for producing a three-dimensional structure of myocardium-like cells having contracting and relaxing functions, intercellular electrical coupling and orientation, which comprises culturing cells which have been obtained from the living heart on a cell culture support having a substrate surface coated with a temperature-responsive polymer whose upper or lower critical solution temperature in water is 0∼80°C, and subsequently:
(1) bringing the temperature of the culture solution to above the upper critical solution temperature or below the lower critical solution temperature;
(2) bringing the cultured cell sheet into close contact with a polymer membrane or mesh;
(3) peeling the cell sheet off together with the polymer membrane or mesh without using a proteolytic enzyme to obtain a myocardium-like cell sheet, and
(4) producing a three-dimensional structure of myocardium-like cells by allowing the thus obtained myocardium-like cell sheet to adhere to another myocardium-like cell sheet, which is adhered to a cell culture support not coated with a temperature-responsive polymer or to a cell culture support coated with a temperature-responsive polymer whose upper or lower critical solution temperature in water is 0∼80°C, and repeating this process as appropriate, thus piling up two or more cell sheets.

2. The process for producing a three-dimensional structure according to claim 1, wherein the three-dimensional structure has been oriented in a specified direction by stretching in the specified direction after it was peeled off from the substrate.

3. The process for producing a three-dimensional structure of myocardium-like cells according to claim 1, wherein the temperature-responsive polymer is poly(N-isopropylacrylamide).

4. The process for producing a three-dimensional structure of myocardium-like cells according to claim 1, wherein the polymer membrane is selected from among a hydrophilized polyvinylidene difluoride membrane, polyurethane, a Spandex mesh and a stockinet-like material.

5. A three-dimensional structure of myocardium-like cells having contracting and relaxing functions, intercellular electrical coupling and orientation that is produced by the process according to any one of claims 1-4.

6. The three-dimensional structure of myocardium-like cells according to claim 5, which is a three-dimensional structure of myocardium-like cells in which tubular cavities are formed of vascular endothelial cells endogenous in said three-dimensional structure of myocardium-like cells and the vascular endothelial cells in the tissue around the buried graft are allowed to grow inward and form tubular cavities, thereby forming blood vessels.

7. The three-dimensional structure of myocardium-like cells according to claim 5, which is suitable for use in the treatment of heart disease and other circulatory organ related diseases or digestive organ related diseases.

8. Use of cells which have been obtained from the living heart for the manufacture of a three-dimensional structure of myocardium-like cells according to any one of claims 5, 6 and 7 for use in the treatment of heart disease and other circulatory organ related diseases or digestive organ related diseases.

9. The three-dimensional structure of myocardium-like cells according to claim 5, for use in a method of treating heart disease and other circulatory organ related diseases or digestive organ related diseases.

## Patentansprüche

1. Verfahren zur Herstellung einer dreidimensionalen Struktur von Myocard-artigen Zellen mit zusammenziehenden und entspannenden Funktionen, interzellulärer elektrischer Kupplung und Orientierung, das umfasst: das Kultivieren von Zellen, die aus dem lebenden Herzen erhalten wurden, auf einer Unterlage mit einer Substratoberfläche, die mit einem auf Temperatur reagierenden Polymer beschichtet ist, dessen obere oder untere kritische Lösungstemperatur in Wasser 0 bis 80°C beträgt und weiterhin:
(1) das Bringen der Temperatur der Kulturlösung über die obere kritische Lösungstemperatur oder unter die untere kritische Lösungstemperatur;
(2) das Bringen des kultivierten Zellblattes in nahen Kontakt mit einer Polymermembran oder einem Netzwerk;
(3) das Abschälen des Zellblattes zusammen mit der Polymermembran oder dem Netzwerk ohne Verwendung eines proteolytischen Enzyms, um ein Myocard-artiges Zellblatt zu erhalten, und
(4) Herstellen einer dreidimensionalen Struktur von Myocard-artigen Zellen durch Ermöglichen, dass das so erhaltene Myocard-artige Zellblatt an ein weiteres Myocard-artiges Zellblatt haftet, das an einer Zellkulturunterlage haftet, die nicht mit einem auf Temperatur reagierenden Polymer beschichtet ist oder an eine Zellkulturunterlage, die mit einem auf Temperatur reagierenden Polymer beschichtet ist, dessen obere oder untere kritische Lösungstemperatur in Wasser 0 bis 80°C beträgt, und Wiederholen dieses Vorgangs wie angemessen, und **dadurch** das Übereinanderschichten von zwei oder mehr Zellblättern.

2. Verfahren zur Herstellung einer dreidimensionalen Struktur gemäß Anspruch 1, wobei die dreidimensionale Struktur in einer festgelegten Richtung orientiert wurde, indem sie in die festgelegte Richtung gestreckt wurde, nachdem sie von dem Substrat abgeschält wurde.

3. Verfahren zur Herstellung einer dreidimensionalen Struktur von Myocard-artigen Zellen gemäß Anspruch 1, wobei das Temperatur reagierende Polymer Poly(N-isopropylacrylamid) ist.

4. Verfahren zur Herstellung einer dreidimensionalen Struktur von Myocard-artigen Zellen gemäß Anspruch 1, wobei die Polymermembran ausgewählt wird zwischen einer hydrophilisierten Polyvinylidendifluoridmembran, Polyurethan, einem Elastan-Netzwerk und einem Trikotartigen Material.

5. Dreidimensionale Struktur von Myocard-artigen Zellen mit zusammenziehenden und entspannenden Funktionen, interzellulärer elektrischer Kupplung und Orientierung, die durch das Verfahren gemäß einem beliebigen der Ansprüche 1 bis 4 hergestellt wurde.

6. Dreidimensionale Struktur von Myocard-artigen Zellen gemäß Anspruch 5, die eine dreidimensionale Struktur von Myocard-artigen Zellen ist, in der röhrenförmige Höhlen von Gefäßendothelzellen gebildet werden, die endogen in der dreidimensionalen Struktur von Myocard-artigen Zellen vorhanden sind und **dadurch**, dass den Gefäßendothelzellen in dem Gewebe um das eingesetzte Transplantat ermöglicht wird, nach innen zu wachsen und röhrenförmige Hohlräume zu bilden, wobei Blutgefäße entstehen.

7. Dreidimensionale Struktur von Myocard-artigen Zellen gemäß Anspruch 5, die geeignet ist zur Verwendung in der Behandlung von einer Herzkrankheit und anderen Kreislauforgan-verwandten Krankheiten oder Verdauungsorgan-verwandten Krankheiten.

8. Verwendung von Zellen, die aus dem lebenden Herzen erhalten wurden, für die Herstellung einer dreidimensionalen Struktur von Myocard-artigen Zellen gemäß einem beliebigen der Ansprüche 5, 6 oder 7, zur Verwendung in der Behandlung von einer Herzkrankheit und anderen Kreislauforgan-verwandten Krankheiten oder Verdauungsorgan-verwandten Krankheiten.

9. Dreidimensionale Struktur von Myocard-artigen Zellen gemäß Anspruch 5, zur Verwendung in einem Verfahren zur Behandlung von einer Herzkrankheit und anderen Kreislauforgan-verwandten Krankheiten oder Verdauungsorgan-verwandten Krankheiten.

## Revendications

1. Procédé pour produire une structure tridimensionnelle de cellules analogues à des cellules myocardiques, ayant des fonctions de contraction et de relaxation, un couplage électrique intercellulaire et une orientation, qui consiste à cultiver des cellules qui ont été obtenues à partir d'un coeur vivant sur un support de culture cellulaire ayant une surface de substrat revêtue d'un polymère sensible à la température dont la température de solution critique supérieure ou inférieure dans l'eau est de 0 à 80°C, et ensuite :
(1) à porter la température de la solution de culture au-delà de la température de solution critique supérieure ou en deçà de la température en solution critique inférieure ;
(2) à mettre la feuille de cellules cultivées en contact étroit avec une membrane ou un treillis polymères ;
(3) à effeuiller de la feuille de cellules conjointement avec la membrane ou le treillis polymères, sans utiliser d'enzyme protéolytique, pour obtenir une feuille de cellules analogues à des cellules myocardiques, et
(4) à produire une structure tridimensionnelle de cellules analogues à des cellules myocardiques en laissant la feuille de cellules analogues à des cellules myocardiques adhérer à une autre feuille de cellules analogues à des cellules myocardiques, qui adhère à un support de culture cellulaire non revêtu d'un polymère sensible à la température ou à un support de culture cellulaire revêtu d'un polymère sensible à la température dont la température de solution critique supérieure ou inférieure dans l'eau est de 0 à 80°C, et en répétant ce procédé selon ce qui est approprié, en empilant ainsi deux feuilles de cellules ou plus.

2. Procédé pour produire une structure tridimensionnelle selon la revendication 1, dans lequel la structure tridimensionnelle a été orientée dans une direction spécifiée par étirage dans la direction spécifiée après l'avoir effeuillée du substrat.

3. Procédé pour produire une structure tridimensionnelle de cellules analogues à des cellules myocardiques selon la revendication 1, dans lequel le polymère sensible à la température est le poly(N-isopropylacrylamide).

4. Procédé pour produire une structure tridimensionnelle de cellules analogues à des cellules myocardiques selon la revendication 1, dans lequel la membrane polymère est choisie parmi une membrane en poly(difluorure de vinylidène) rendu hydrophile, le polyuréthane, un treillis en Spandex et un matériau de type jersey tubulaire.

5. Structure tridimensionnelle de cellules analogues à des cellules myocardiques ayant des fonctions de contraction et de relaxation, un couplage électrique intercellulaire et une orientation, qui est produite par le procédé selon l'une quelconque des revendications 1 à 4.

6. Structure tridimensionnelle de cellules analogues à des cellules myocardiques selon la revendication 5, qui est une structure tridimensionnelle de cellules analogues à des cellules myocardiques dans laquelle sont formées des cavités tubulaires de cellules endothéliales vasculaires endogènes dans ladite structure tridimensionnelle de cellules analogues à des cellules myocardiques et les cellules endothéliales vasculaires dans le tissu aux alentours du greffon enfoui sont laissées à croître vers l'intérieur et forment des cavités tubulaires, en formant ainsi des vaisseaux sanguins.

7. Structure tridimensionnelle de cellules analogues à des cellules myocardiques selon la revendication 5, qui convient pour une utilisation dans le traitement de maladies cardiaques et d'autres maladies liées à un organe circulatoire ou des maladies liées à un organe digestif.

8. Utilisation de cellules qui ont été obtenues à partir d'un coeur vivant, pour la fabrication d'une structure tridimensionnelle de cellules analogues à des cellules myocardiques selon l'une quelconque des revendications 5, 6 et 7, destinée à être utilisée dans le traitement de maladies cardiaques et d'autres maladies liées à un organe circulatoire ou des maladies liées à un organe digestif.

9. Structure tridimensionnelle de cellules analogues à des cellules myocardiques selon la revendication 5, destinée à être utilisée dans une méthode de traitement d'une maladie cardiaque et d'autres maladies liées à un organe circulatoire ou des maladies liées à un organe digestif.
